# EUROPÄISCHE PATENTANMELDUNG

(11) **EP 1 055 393 A2**
(43) Veröffentlichungstag der Anmeldung: **29.11.2000**
(21) Anmeldenummer: 00890158.9
(22) Anmeldetag: 18.05.2000
(51) Int. Cl.: A61B 5/022

(54) **Verfahren und Vorrichtung zur Ermittlung eines von respiratorischen Messdaten abhängigen Indikators**

(30) Priorität: 25.05.1999 AT 93299
(71) Anmelder: AVL Medical Instruments AG, 8207 Schaffhausen (CH)
(72) Erfinder: Pessenhofer, Herfried, Prof.Dipl.-Ing. Dr., 8043 Graz (AT)
(74) Vertreter: Babeluk, Michael, Dipl.-Ing. Mag.

(57) **Zusammenfassung**

Bei einem Verfahren zur Ermittlung eines von respiratorischen Meßdaten abhängigen Indikators, welcher den Übergang vom aeroben zum anaeroben Stoffwechsel einer Person definiert, wobei die Person einer körperlichen Belastung unterworfen wird, wird die O₂- und CO₂-Konzentration direkt im Atemgasstrom der Person oder in einem davon abgeleiteten Teilstrom gemessen. Der zeitliche Verlauf der Konzentrationswerte bzw. diskrete Konzentrationswerte werden innerhalb zumindest einer Respirationsperiode erfaßt, und der den aerobanaeroben Übergang definierende Indikator unter Verwendung eines mathematischen Modells aus dem zeitlichen Verlauf der Konzentrationswerte bzw. aus den diskreten Konzentrationswerten abgeleitet.

## Beschreibung

Die Erfindung betrifft ein Verfahren zur Ermittlung eines von respiratorischen Messdaten abhängigen Indikators, welcher den Übergang vom aeroben zum anaeroben Stoffwechsel einer Person definiert, wobei die Person einer körperlichen Belastung unterworfen wird, sowie eine Vorrichtung zur Durchführung des Verfahrens.

Die körperliche Leistungsfähigkeit eines Organismus stellt eine integrative und unscharf beschriebene Größe dar, die die Fähigkeit, physische Belastungen zu tolerieren, quantifizieren soll. Durch eine körperliche Belastung werden jedoch mehrere Subsysteme des Organismus in unterschiedlicher und vom Trainingszustand abhängiger Ausprägung beansprucht. Die wichtigsten dieser Subsysteme sind - neben den neuromuskulären Systemen zur Bewerkstelligung des Bewegungsablaufs - das Sauerstofftransportsystem (Atmung, Diffusion der Atemgase, Sauerstoffbindung ans Hämoglobin, Herz-Kreislauf-System) und das Stoffwechselsystem des arbeitenden Skelettmuskels.

Da beim gesunden Menschen das Stoffwechselsystem des Skelettmuskels das leistungslimitierende System darstellt, wird in der praktischen Leistungsphysiologie dem Muskelstoffwechsel die primäre Aufmerksamkeit zugewandt.

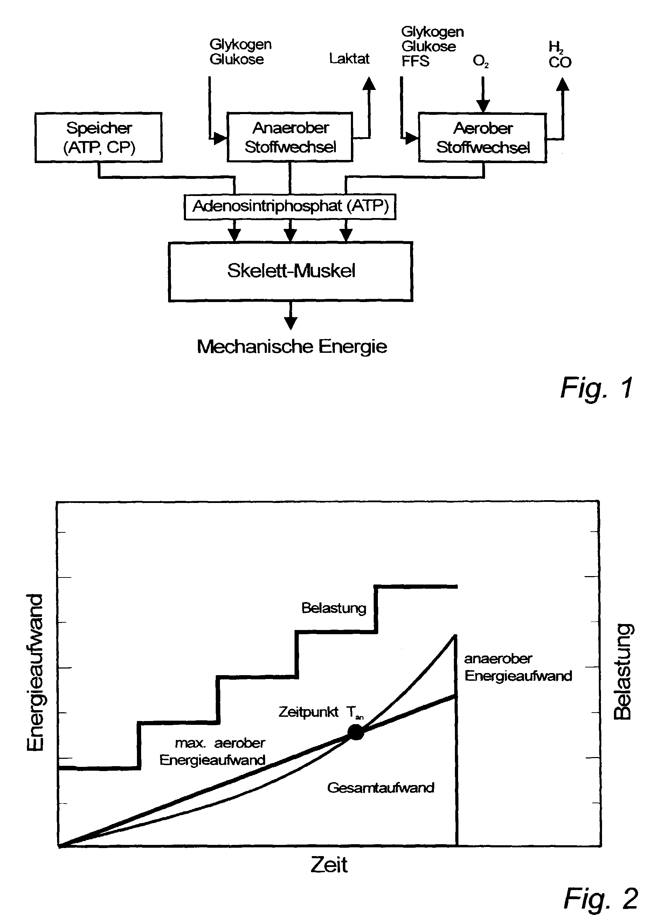

Zur Erbringung körperlicher Leistung muss der Muskel auf einen Pool von Adenosintriphosphat (ATP) als unmittelbare Energiequelle zurückgreifen (siehe Fig. 1). Für dessen Resynthese stehen - Kurzzeitbelastungen ausgenommen, für die Phosphatspeicher herangezogen werden - zwei Stoffwechselvarianten zur Verfügung, die nach den jeweiligen Anforderungen seitens der Belastungsintensität und der Belastungsdauer in Anspruch genommen werden. Der wichtigste und energetisch günstigste Weg ist jener des aeroben Stoffwechsels, bei dem die Substrate Glykogen und Glukose, aber auch freie Fettsäuren bei ausreichender Sauerstoffzufuhr über Atmung und Kreislauf zu Kohlendioxid und Wasser abgebaut werden.

Der aerobe Stoffwechselweg kann jedoch nur mäßig hohe Energieflussraten bereitstellen, daher wird bei hohen geforderten Leistungen (auch am Beginn der Körperarbeit) die zweite Stoffwechselvariante, der anaerobe Weg, zusätzlich aktiviert. Dieser Mechanismus der Energiebereitstellung greift ebenso auf Glykogen und Glukose als Substrat zurück, liefert jedoch ein Endprodukt, das Laktat. Dieses wird im Normalfall rascher gebildet als es abgebaut werden kann und akkumuliert daher im Organismus. Gleichzeitig werden Wasserstoffionen produziert, so dass es durch die mit dem Laktatanstieg verbundene Azidose zum Abbruch der Tätigkeit wegen muskulärer Erschöpfung kommt.

Für ein Gesundheitstraining, z.B. Ausdauertraining, stellt eine körperliche Belastung, bei der primär der aerobe Stoffwechselweg beansprucht wird, die ideale und erstrebenswerte Form dar, da einerseits eine Ökonomisierung des Herz-Kreislaufsystems erreicht wird und anderseits die Mobilisierung des Stoffwechsels und der Fettabbau begünstigt werden.

In der Leistungsphysiologie wird häufig von der in Fig. 2 dargestellten idealisierten Vorstellung ausgegangen, dass auf dem Wege der biochemischen Energiegewinnung der durch das Belastungsprotokoll vorgegebene Energieaufwand (unter Einbeziehung des mechanischen Wirkungsgrades) abgedeckt wird, solange die geforderte Belastung geringer als die individuell festgelegte Maximalbelastung ist. Beim dargestellten Belastungsprotokoll nimmt der Energiegesamtaufwand nach einer annähernd quadratischen Funktion zu.

Der maximal über den aeroben Stoffwechsel abdeckbare Anteil wird durch eine Gerade dargestellt, deren Steigung von der maximalen aeroben Kapazität des Probanden (u.a. eine Funktion des Trainingszustands), d.h. der maximalen aeroben Leistungsfähigkeit des Probanden abhängt.

Überschreitet der Gesamtaufwand den maximal aerob erbringbaren Aufwand, so muss der Differenzanteil zum Gesamtaufwand über den anaeroben Stoffwechsel bereitgestellt werden.

Jener Zeitpunkt Tₐₙ, der Zeitpunkt des aerob-anaeroben Übergangs, bei dem die aerobe Energiebereitstellung überschritten wird, wird nun ― bei definiertem Belastungsprotokoll ― in Belastungseinheiten (z.B. Watt) angegeben.

In der Praxis ist jedoch der aerob-anaerobe Übergang nicht exakt durch einen Zeitpunkt bzw. Belastungswert angebbar, so dass man häufig einen Übergangsbereich definiert, an dessen Untergrenze (aerobe Schwelle) die Umstellung des Energiestoffwechsels beginnt und an dessen Obergrenze (anaerobe Schwelle) abgeschlossen ist.

Da die Belastungsintensität, bei der der Organismus vom energetisch günstigeren aeroben Stoffwechsel zum anaeroben übergeht, vom aktuellen Trainingszustand und damit von der Leistungsfähigkeit abhängt, werden im Rahmen von leistungsphysiologischen Untersuchungen bzw. Leistungstests verschiedene Indikatoren definiert, die sich an diversen physiologischen Größen bzw. Variablen der beteiligten Subsysteme des Organismus und deren Veränderung im Rahmen von standardisierten Belastungsprotokollen orientieren.

Je nach ihrer Zuordnung zu den verschiedenen Subsystemen spricht man von folgenden Indikatoren:
― Laktatorientierte Indikatoren
― Herzfrequenzorientierte Indikatoren
― Respiratorisch orientierte Indikatoren.

Im Falle der laktatorientierten Indikatoren wird die Laktatkonzentration im Blut als Kenngröße herangezogen. Um einen Zahlenwert für die Leistungsfähigkeit zu erhalten, wird empirisch über Ergometertests, die ein standardisiertes Stufenprotokoll verwenden, der Funktionszusammenhang zwischen Laktatkonzentration und Ergometerleistung bestimmt. Die Ergometerleistung an zwei definierten Laktatkonzentrationen, nämlich 2 mmol/l und 4 mmol/l wird als Kenngröße für die Leistungsfähigkeit herangezogen, der 2 mmol/l-Wert wird dabei als aerobe Schwelle, der 4 mmol/l-Wert als anaerobe Schwelle, der Zwischenbereich als aerob-anaerober Übergang bezeichnet.

Bei den herzfrequenzorientierten Kriterien werden entweder Absolutwerte der Herzfrequenz z.B. 60% der maximalen Herzfrequenz, oder Spezifika des Zeitverlaufs der Herzfrequenz (Unstetigkeiten bzw. Knickpunkte) bei einem ansteigenden Belastungsprotokoll als Indikator für den Übergang vom aeroben auf den anaeroben Stoffwechselweg herangezogen. Die bekannteste Form dieser Testverfahren stellt der sogenannte CONCONI-Test dar.

Die respiratorisch orientierten Kriterien basieren auf der physiologischen Tatsache, dass bei Inanspruchnahme des anaeroben Stoffwechsels neben Laktat auch Wasserstoffionen gebildet werden, die zu einer metabolischen Azidose führen. Die Wasserstoffionen werden durch das Bikarbonat-Puffersystem des Blutes abgepuffert, wobei CO₂ frei wird. Dieses wird über das Respirationssystem abgeatmet. Zusätzlich wird ― gesteuert über die Atemregulation ― eine kompensatorische Hyperventilation initiiert, so dass es zu einem verstärkten Abatmen von Kohlendioxid kommt. Man setzt daher häufig die Sauerstoffaufnahme mit der Kohlendioxidabgabe in Beziehung (z. B. durch Berechnung der sogenannten ^{"}respiratory exchange ratio ― RER") und versucht auf diese Weise Indikatoren für den Übergang vom aeroben auf den anaeroben Stoffwechsel abzuleiten.

Die am häufigsten in der Leistungsdiagnostik herangezogenen Indikatoren sind die laktatorientierten, die sogenannten Laktatschwellen. Ihr Vorteil liegt in der messtechnisch präzisen Bestimmbarkeit, ihr Nachteil ist die notwendige Blutabnahme aus dem hyperämisierten Ohrläppchen und die instrumentelle Ausrüstung zur Analytik.

Herzfrequenzorientierte Indikatoren sind einfach und nichtinvasiv zu bestimmen, besitzen jedoch aufgrund der indirekten Abbildung von Kenngrößen des Muskelstoffwechsels auf eine kardiovaskuläre Größe mangelnde Genauigkeit und Reproduzierbarkeit.

Respiratorisch orientierte Indikatoren besitzen den Vorteil der direkten Zuordnung zum Muskelstoffwechsel (metabolische Azidose), ihre Bestimmung ist jedoch nach dem bislang üblichen Verfahren der Ergospirometrie an eine umfangreiche apparative Ausrüstung gebunden.

Beispielsweise ist aus der US-A 5 297 558 ein Algorithmus zur Bestimmung eines Indikators für den "fat burning point" bekannt. Dieser Punkt entspricht im wesentlichen dem oben erwähnten aerob-anaeroben Übergang, und wird konventionell auf respiratorischer Basis bestimmt. Dabei wird mittels einer aus der US-A 4 463 764 bekannten Ergospirometrieanlage basierend auf Sauerstoffaufnahme (Volumen/Zeit) und Kohlendioxidabgabe (Volumen/Zeit) der sogenannten respiratorische Quotient (RER) bestimmt. Die Herzfrequenz bei jener Belastungsintensität, bei der dieser RER den Zahlenwert von 0,9 überschreitet, wird als Indikatorgröße herangezogen und prozentuelle Auf- bzw. Abschläge auf diesen Herzfrequenzwert bilden den - nun auf Herzfrequenz-Basis angegebenen - optimalen Intensitätsbereich für das Training. Nachteilig dabei ist die dafür notwendige Bestimmung des Atemzeitvolumens. Weiters weist die Bestimmung des Atemzeitvolumens aufgrund der erforderlichen Atemmasken bzw. Mundstücke (Dichtigkeitsprobleme) nur geringe Genauigkeit auf, wobei die Verwendung dicht sitzender Masken bzw. das Atmen über Mundstücke meist als unangenehm empfunden wird.

Aus der US-A 5 782 772 A ist weiters ein Verfahren sowie eine Vorrichtung zur Bestimmung der individuellen anaeroben Schwelle bekannt, wobei als Primärgrößen das Atemzeitvolumen V_{E}, die Kohlendioxydabgabe V_{CO2} und die Sauerstoffaufnahme V_{O2} bestimmt werden. Alle genannten Größen sind sogenannte ^{"}Flussgrößen" mit der physikalischen Dimension Volumen/Zeit (Einheit: 1/min, ml/min, etc.). Die Bestimmung dieser Größen setzt in allen Fällen eine gasdichte Atemmaske voraus, wobei die oben zur US-A 4 463 764 angeführten Nachteile auftreten. Zur Ermittlung der Kohlendioxydabgabe und der Sauerstoffaufnahme müssen die jeweiligen Gaskonzentrationen gemessen werden.

Aufgabe der Erfindung ist es, ein Verfahren bzw. eine Vorrichtung zur Durchführung des Verfahrens vorzuschlagen, um eine einfache Bestimmung des aerob-anaeroben Übergangs auf der Basis respiratorischer Kriterien zu ermöglichen, wobei für den Probanden unangenehme Begleitumstände bei Anwendung des Verfahrens bzw. der Vorrichtung weitgehend vermieden werden sollen.

Diese Aufgabe wird erfindungsgemäß dadurch gelöst, dass die O₂- und CO₂ - Konzentration direkt im Atemgasstrom der Person oder in einem davon abgeleiteten Teilstrom gemessen wird, wobei der zeitliche Verlauf der Konzentrationswerte innerhalb zumindest einer Respirationsperiode erfasst wird, sowie dass der den aerob-anaeroben Übergang definierende Indikator unter Verwendung eines mathematischen Modells aus dem zeitlichen Verlauf der Konzentrationswerte abgeleitet wird.

Alternativ dazu können zu vorbestimmten Zeitpunkten (beispielsweise unter Zuhilfenahme einer Thermistor-Sonde) im Verlauf zumindest einer Respirationsperiode diskrete O₂- und CO₂- Konzentrationswerte gemessen werden, wobei der den aerob-anaeroben Übergang definierende Indikator unter Verwendung eines mathematischen Modells aus den diskreten O₂-und CO₂- Konzentrationswerten abgeleitet wird.

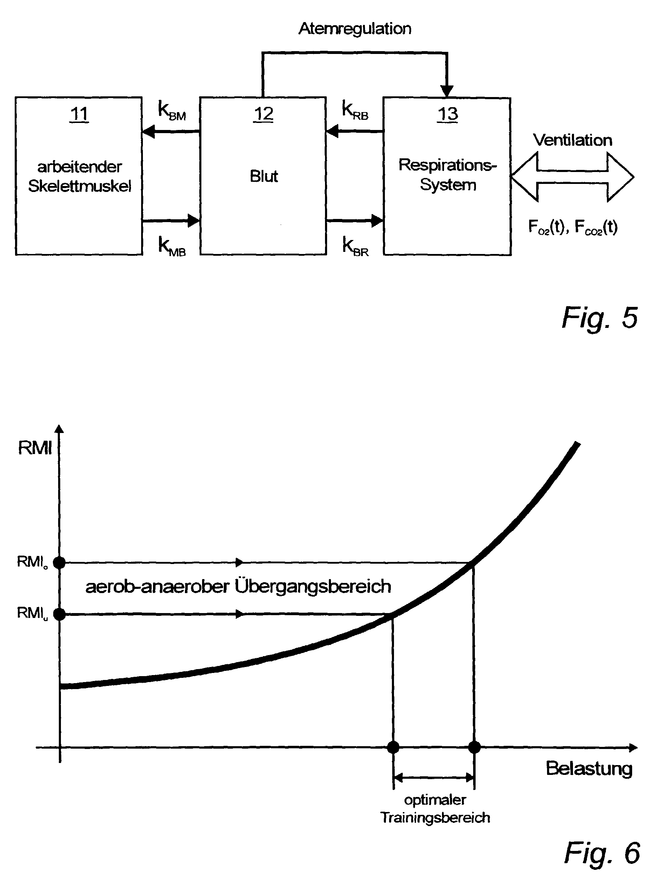

Im Gegensatz zur bislang üblichen Volumenbestimmung wird ausschließlich auf die Zeitverläufe bzw. punktuelle Bestimmungen der Konzentrationswerte von Sauerstoff und Kohlendioxid im Verlauf zumindest einer Respirationsperiode Bezug genommen. Aus diesen wird unter Verwendung eines z. B. empirischen oder aus physiologisch-medizinischen Basisinformationen ableitbaren mathematischen Modells (Siehe z.B. Fig. 5 und 6) ein Indikatorwert berechnet.

Der Indikator, der den Übergang vom aeroben zum anaeroben Stoffwechsel charakterisiert, kann sowohl als Richtgröße für die Steuerung oder Regelung der Intensität eines Gesundheitstrainings im Rahmen von stationären Einrichtungen, beispielsweise Fitnesszentren oder von individuellen Trainingsmaßnahmen im freien Feld, z. B. Jogging, herangezogen werden, als auch für Zwecke der Leistungsdiagnostik, nämlich zur Bestimmung der körperlichen Leistungsfähigkeit. In letzterem Fall muss zusätzlich zu den Konzentrationswerten auch die von der einer körperlichen Belastung unterworfenen Person erbrachte Leistung gemessen werden, um den errechneten Indikator einer bestimmten Leistung zuordnen zu können.

Eine erfindungsgemäße Vorrichtung zur Ermittlung eines Indikators, welcher den Übergang vom aeroben zum anaeroben Stoffwechsel einer Person definiert, zeichnet sich dadurch aus, dass direkt im Atemgasstrom oder in einem vom Atemgasstrom gewonnenen Teilstrom positionierbare Sensoren zur Messung der O₂- und der CO₂-Konzentration vorgesehen sind, welche geeignet sind, den zeitlichen Verlauf der Konzentrationswerte oder diskrete Konzentrationswerte innerhalb zumindest einer Respirationsperiode zu erfassen. Es wird somit entweder quasi-kontinuierlich oder in Form diskreter Werte (z.B. Minimal- oder Maximalwerte) gemessen. Vorteilhafterweise kann somit direkt im Atemgasstrom gemessen werden, wobei die Sensoren, beispielsweise an einer Halterung ähnlich einer Freisprecheinrichtung vor den Atemorganen positioniert sind, oder es kann über eine an den Atemorganen der Person lose anliegende, masken- oder trichterähnliche Vorrichtung ein Teilstrom aus dem Atemgasstrom gewonnen werden. In letzterem Fall kann zwischen der maskenähnlichen Vorrichtung zur Gewinnung des Teilstromes und den Sensoren zur Messung der O₂- und der CO₂-Konzentration eine Einrichtung zur Trocknung der Atemgase vorgesehen sein.

Es ist von Vorteil, wenn der zeitliche Verlauf der O₂- und der CO₂-Konzentration über mehrere Respirationsperioden bei gleichbleibender Belastung ermittelt und das Ergebnis vorzugsweise digitalisiert gespeichert wird.

In einer vorteilhaften Ausführungsvariante der Erfindung ist vorgesehen, dass aus dem - zeitlichen Verlauf der Konzentrationswerte für O₂ und CO₂ die endexspiratorischen Werte für O₂ und CO₂ berechnet und für die Ableitung des Indikators herangezogen werden.

Weiters ist erfindungsgemäß vorgesehen, dass zusätzlich die Belastung bzw. Leistung und die Herzfrequenz der Person gemessen und als diagnostische Größen angezeigt oder als Steuergröße verwendet werden, bzw. dass im mathematischen Modell Größen berücksichtigt werden, welche die Mischungscharakteristika des Respirationstraktes, die Kompartimentierung des Muskelstoffwechsels und die Puffersysteme des Blutes und/oder die Gastransport- und Signalcharakteristika des Meßsystems beschreiben.

Erfindungsgemäß können auch Formkriterien des exspiratorischen Anteils des zeitlichen Verlaufs der Konzentrationswerte von O₂ und CO₂ zur Bestimmung des Indikators verwendet werden. Aussagekräftig ist somit nicht nur der errechnete endexspiratorische Konzentrationswert, sondern auch die Form des zeitlichen Verlaufs (Anstieg, Unstetigkeiten, etc..) in der exspiratorischen Phase.

Die Erfindung wird im folgenden anhand von Zeichnungen näher erläutert. Es zeigen:
- Fig. 1: eine graphische Darstellung der für die Energiebereitstellung betrachteten Stoffwechselvarianten,
- Fig. 2: das Diagramm eines Belastungsprotokolls,
- Fig. 3: die erfindungsgemäße Vorrichtung zum Teil in schematischer Darstellung,
- Fig. 4: den zeitlichen Verlauf der O₂- und der CO₂ -Konzentrationswerte in der Inspirations- und der Exspirationsphase mehrerer Respirationsperioden (Tᵣₑₛₚ),
- Fig. 5: die graphische Darstellung eines mathematischen Modells zur Bestimmung eines Indikatorwertes sowie
- Fig. 6: den Verlauf des respiratorisch-metabolischen Indikators (RMI) als Funktion der Belastung.

Die in den Fig. 1 und 2 graphisch dargestellte allgemeine Problematik wurde bereits eingangs ausführlich diskutiert.

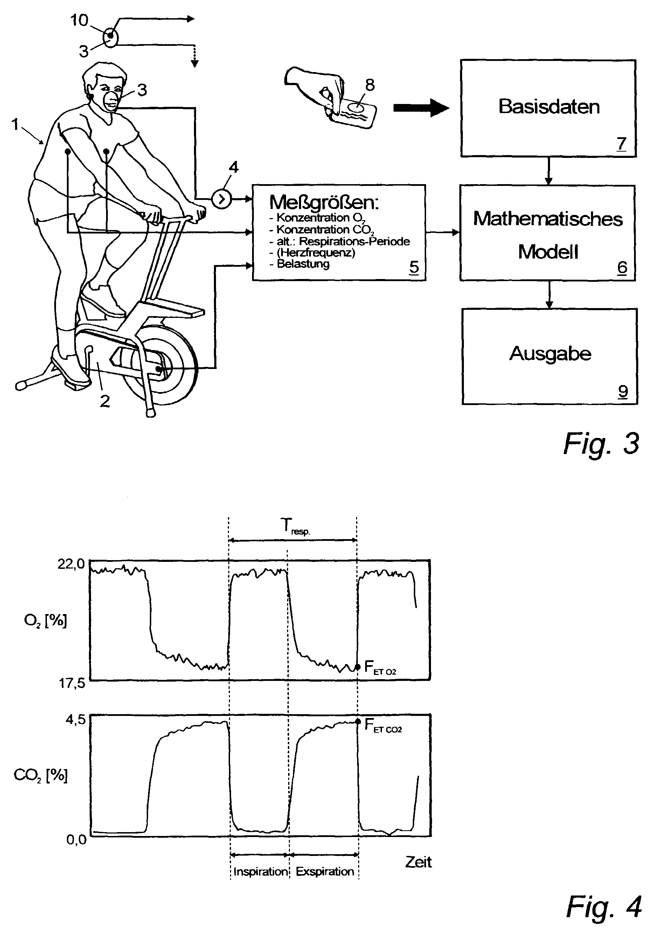

Fig. 3 zeigt eine erfindungsgemäße Vorrichtung zur Ermittlung eines von respiratorischen Messdaten abhängigen Indikators, welcher den Übergang vom aeroben zum anaeroben Stoffwechsel einer Person 1 definiert. Die Person ist einer körperlichen Belastung, beispielsweise in Form eines Fahrradergometers 2, unterworfen. Über eine lose anliegende maskenähnliche Vorrichtung 3 wird der respiratorische Gaswechsel des Probanden erfasst und mittels einer Pumpe 4 ein konstanter Teilstrom abgesaugt. Dieser wird nach eventueller Trocknung einer Messeinheit 5 mit Gasanalysatoren mit geringer Einstellzeit für Sauerstoff und Kohlendioxid zugeführt. Weiters werden der Messeinheit 5 Daten über die aktuelle Belastung des Probanden bzw. die von ihm erbrachte Leistung und die Herzfrequenz der Person zugeführt. Die Daten werden in einer Prozessoreinheit 6 auf der Basis eines mathematischen Modells verarbeitet, wobei über eine weitere Einheit 7, beispielsweise mittels einer Magnetkarte 8, personenbezogene Basisdaten, wie z. B. Alter, Gewicht, Größe, Geschlecht und Trainingszustand des Probanden eingegeben werden können. Die errechneten Daten werden in einer Anzeige- oder Ausgabeeinheit 9 dargestellt. Alternativ kann in der maskenähnlichen Vorrichtung 3 bzw. direkt in der Nase des Probanden ein Thermistor-Sensor 10 zur Erfassung des Atemzyklus vorgesehen sein.

Die erfindungsgemäße Vorrichtung kann auch als portables, mobiles Gerät ausgebildet sein, welches beim Training, beispielsweise beim Jogging mitgeführt bzw. am Körper befestigt wird.

Die in Fig. 4 dargestellten Zeitverläufe der Konzentrationen von O₂ und CO₂ werden über mehrere Respirationsperioden Tᵣₑₛₚ(ca. 10 Perioden) aufgezeichnet und digital abgespeichert. Die endexspiratorischen Werte für O₂ und CO₂ sind mit F_{ET O2} bzw. mit F_{ET CO2} bezeichnet.

Aus den gespeicherten Zeitfunktionen werden über ein mathematisches Modell Indikatoren für den Status des Muskelstoffwechsels identifiziert. Dieses Modell kann entsprechend der Kompartimentierung der beteiligten physiologischen Systeme aus drei Submodellen (siehe Fig. 5) bestehen, nämlich dem arbeitenden Skelettmuskel 11, dem Blutkompartment 12 und dem Respirations-System 13. Im Submodell 11 wird ein funktionaler Zusammenhang zwischen Wasserstoffionen-Produktion auf der einen Seite und Laktatproduktion, Belastung, Sauerstoffaufnahme, Herzzeitvolumen und utilisierten Substraten auf der anderen Seite etabliert. Im Submodell 12 wird ein Funktionsansatz für das Bikarbonat-Puffersystem des Blutes erstellt und die CO₂-Produktion aufgrund der Aktivität dieses Systems ermittelt. Im Submodell 13 wird ein formaler Zusammenhang zwischen den Gaskonzentrationswerten im Blut und den Zeitverläufen der Atemgas-Konzentrationswerte bestimmt, wobei in diesem formalen Ansatz anthropometrische Daten der Person (Körpergröße, Körpergewicht, Alter) sowie Charakteristika des Respirations-Systems (Resistance, funktionelle Residualkapazität etc.) und die Belastungsintensität eingehen. Der Effekt der Atemregulation auf den CO₂-Konzentrationsverlauf im Atemgasstrom wird über einen nichtlinearen Funktionsansatz modelliert. Das Gesamtmodell, das in Form eines Differentialgleichungs-Systems in Verbindung mit algebraischen Gleichungen formuliert ist, wird auf der Basis der experimentell bestimmten Konzentrations-Zeitverläufe für O₂ und CO₂ sowie der als Parameter vorliegenden anthropometrischen Daten identifiziert.

Auf der Basis der identifizierten Modellparameter wird ein Kriteriumswert errechnet, der ein Abbild der metabolischen Situation des arbeitenden Skelettmuskels, repräsentiert durch Laktat- bzw. Wasserstoffionen-Produktion, darstellt. In diesem Indikator sind auch die unterschiedlichen Dynamiken der Änderung von metabolischen und respiratorischen Variablen berücksichtigt.

Für den resultierenden respiratorisch-metabolische Indikator (RMI) als Funktion der Belastung können nun ― wie in Fig. 6 dargestellt ― Schranken für den aerob-anaeroben Übergangsbereich nach empirischen Kriterien definiert werden (RMIᵤ und RMIₒ).

Die ermittelten Indikatoren können in Beziehung zur Belastungsintensität bzw. zur Herzfrequenz bei der jeweiligen Belastung gesetzt werden und als diagnostische Angaben für die Leistungsfähigkeit des jeweiligen Probanden sowie als Steuergrößen für das Ausdauertraining herangezogen werden.

## Patentansprüche

1. Verfahren zur Ermittlung eines von respiratorischen Messdaten abhängigen Indikators, welcher den Übergang vom aeroben zum anaeroben Stoffwechsel einer Person definiert, wobei die Person einer körperlichen Belastung unterworfen wird, **dadurch gekennzeichnet**, dass die O₂- und CO₂-Konzentration direkt im Atemgasstrom der Person oder in einem davon abgeleiteten Teilstrom gemessen wird, wobei der zeitliche Verlauf der Konzentrationswerte innerhalb zumindest einer Respirationsperiode erfasst wird, sowie dass der den aerob-anaeroben Übergang definierende Indikator unter Verwendung eines mathematischen Modells aus dem zeitlichen Verlauf der Konzentrationswerte abgeleitet wird.

2. Verfahren nach Anspruch 1, **dadurch gekennzeichnet**, dass der zeitliche Verlauf der O₂- und der CO₂-Konzentration über mehrere Respirationsperioden bei gleichbleibender Belastung gemittelt und das Ergebnis vorzugsweise digitalisiert gespeichert wird.

3. Verfahren nach Anspruch 1 oder 2, **dadurch gekennzeichnet**, dass aus dem zeitlichen Verlauf der Konzentrationswerte für O₂ und CO₂ die endexspiratorischen Werte für O₂ und CO₂ berechnet und für die Ableitung des Indikators herangezogen werden.

4. Verfahren nach einem der Patentansprüche 1 bis 3, **dadurch gekennzeichnet**, dass zusätzlich die Belastung bzw. Leistung und die Herzfrequenz der Person gemessen und als diagnostische Größen angezeigt und/oder als Steuergröße verwendet werden.

5. Verfahren nach einem der Ansprüche 1 bis 4, **dadurch gekennzeichnet**, dass im mathematischen Modell Größen berücksichtigt werden, welche die Mischungscharakteristika des Respirationstraktes, die Kompartimentierung des Muskelstoffwechsels, die Puffersysteme des Blutes und/oder die Gastransport- und Messcharakteristika des Meßsystems beschreiben.

6. Verfahren nach einem der Ansprüche 1 bis 5, **dadurch gekennzeichnet**, dass Formkriterien des in- bzw. exspiratorischen Anteils des zeitlichen Verlaufs der Konzentrationswerte von O₂ und CO₂ zur Bestimmung des Indikators verwendet werden.

7. Verfahren nach einem der Ansprüche 1 bis 6, **dadurch gekennzeichnet**, dass in das mathematische Modell personenbezogene Daten, wie z. B. Alter, Gewicht, Größe, Geschlecht, Trainingszustand u. s. w. einbezogen werden.

8. Verfahren zur Ermittlung eines von respiratorischen Messdaten abhängigen Indikators, welcher den Übergang vom aeroben zum anaeroben Stoffwechsel einer Person definiert, wobei die Person einer körperlichen Belastung unterworfen wird, **dadurch gekennzeichnet**, dass zu vorbestimmten Zeitpunkten im Verlauf zumindest einer Respirationsperiode diskrete O₂- und CO₂-Konzentrationswerte direkt im Atemgasstrom der Person oder in einem davon abgeleiteten Teilstrom gemessen werden, sowie daß der den aerobanaeroben Übergang definierende Indikator unter Verwendung eines mathematischen Modells aus den diskreten O₂- und CO₂-Konzentrationswerten abgeleitet wird.

9. Vorrichtung zur Ermittlung eines von respiratorischen Meßdaten abhängigen Indikators, welcher den Übergang vom aeroben zum anaeroben Stoffwechsel einer Person (1) definiert, wobei die Person (1) einer körperlichen Belastung unterworfen ist, mit einer Prozessoreinheit (6) zur Verarbeitung der respiratorischen Meßdaten, sowie einer Anzeige und/oder Ausgabeeinheit (9), **dadurch gekennzeichnet**, daß direkt im Atemgasstrom oder in einem vom Atemgasstrom gewonnenen Teilstrom positionierbare Sensoren zur Messung der O₂- und der CO₂-Konzentration vorgesehen sind, welche geeignet sind, den zeitlichen Verlauf der Konzentrationswerte oder diskrete Konzentrationswerte innerhalb zumindest einer Respirationsperiode zu erfassen.

10. Vorrichtung nach Anspruch 9, **dadurch gekennzeichnet**, daß zur Gewinnung des Teilstroms eine an den Atemorganen der Person (1) lose anliegende, maskenähnliche Vorrichtung (3) vorgesehen ist.

11. Vorrichtung nach Anspruch 10, **dadurch gekennzeichnet**, daß in der maskenähnlichen Vorrichtung (3) ein Thermistor-Sensor (10) zur Bestimmung der Respirationsperiode vorgesehen ist.

12. Vorrichtung nach Anspruch 10 oder 11, **dadurch gekennzeichnet**, dass zwischen der maskenähnlichen Vorrichtung (3) zur Gewinnung des Teilstromes und den Sensoren zur Messung der O₂- und CO₂-Konzentration eine Einrichtung zur Trocknung der Atemgase vorgesehen ist.
